# EUROPEAN PATENT APPLICATION

(11) **EP 1 566 181 A1**
(43) Date of publication of application: **24.08.2005**
(21) Application number: 03812336.0
(22) Date of filing: 28.11.2003
(51) Int. Cl.: A61K 38/17, A61P 1/00, A61P 3/12, A61P 9/00, A61P 15/10, A61P 21/04, A61P 25/00, A61P 25/02, A61P 25/14, A61P 25/16, A61P 25/18, A61P 25/22, A61P 25/24, A61P 25/28, A61P 27/06, A61P 39/02, A61P 43/00

(54) **NOVEL MEDICAMENT FOR AMELIORATING NEUROTRANSMISSION DYSFUNCTION DISEASES**

(30) Priority: 29.11.2002 JP 2002348714
(71) Applicant: Juridical Foundation, The Chemo-Sero-Therapeutic Research Institute, Kumamoto 860-8568 (JP)
(72) Inventor: KAWAMURA, Ryoichi, Kumamoto-shi, Kumamoto 860-0881 (JP); NARUSE, Takeshi, Kikuchi-gun, Kumamoto 861-1102 (JP); HIRASHIMA, Masaki, Kikuchi-gun, Kumamoto 861-1102 (JP); KAMINAKA, Kazuyoshi, Kikuchi-gun, Kumamoto-ken 869-1101 (JP); MATSUDA, Junichi, Kumamoto-shi, Kumamoto 862-8002 (JP); MAEDA, Hiroaki, Kumamoto-shi, Kumamoto 860-0076 (JP); NODA, Mami, Fukuoka-shi, Fukuoka 819-0044 (JP); WADA, Keiji, Kodaira-shi, Tokyo 187-0031 (JP)
(74) Representative: Vossius & Partner
(86) International application number: PCT/JP2003/015227
(87) International publication number: WO 2004/050114

(57) **Abstract**

A novel medicament for ameliorating neurotransmission dysfunction diseases is provided. A medicament for ameliorating neurotransmission dysfunction diseases comprising as a main active ingredient preferably a selenocysteine-containing protein such as Selenoprotein P or a selenocysteine-containing peptide that consists of said protein or a series of said peptides. A medicament suited for ameliorating neurotransmission dysfunction diseases caused by various pathological conditions is provided.

SEQUENCE LISTING
<110> JURIDICAL FOUNDATION THE CHEMO-SERO-THERAPEUTIC RESEARCH INSTITUTE
<120> Novel Remedies for Diseases of abnormal neurotransmission
<130> 664194
<150> JP 2002-348714
<151> 2002-11-29
<160> 5

<210> 1
<211> 362
<212> PRT
<213> Homo sapiens
<220>
<223> Xaa represents selenocysteine
<400> 1
Glu Ser Gln Asp Gln Ser Ser Leu Cys Lys Gln Pro Pro Ala Trp
1               5                   10                  15
Ser Ile Arg Asp Gln Asp Pro Met Leu Asn Ser Asn Gly Ser Val
16 20 25 30
Thr Val Val Ala Leu Leu Gln Ala Ser Xaa Tyr Leu Cys Ile Ile
31 35 40 45
Glu Ala Ser Lys Leu Glu Asp Leu Arg Val Lys Leu Lys Lys Glu
46 50 55 60
Gly Tyr Ser Asn Ile Ser Tyr Ile Val Val Asn His Gln Gly Ile
61 65 70 75
Ser Ser Arg Leu Lys Tyr Thr His Leu Lys Asn Lys Val Ser Glu
76 80 85 90
His Ile Pro Val Tyr Gln Gln Glu Glu Asn Gln Thr Asp Val Trp
91 95 100 105

Thr Leu Leu Asn Gly Ser Lys Asp Asp Phe Leu Ile Tyr Asp Arg
106 110 115 120
Cys Gly Arg Leu Val Tyr His Leu Gly Leu Pro Phe Ser Phe Leu
121 125 130 135
Thr Phe Pro Tyr Val Glu Glu Ala Ile Lys Ile Ala Tyr Cys Glu
136 140 145 150
Lys Lys Cys Gly Asn Cys Ser Leu Thr Thr Leu Lys Asp Glu Asp
151 155 160 165
Phe Cys Lys Arg Val Ser Leu Ala Thr Val Asp Lys Thr Val Glu
166 170 175 180
Thr Pro Ser Pro His Tyr His His Glu His His His Asn His Gly
181 185 190 195
His Gln His Leu Gly Ser Ser Glu Leu Ser Glu Asn Gln Gln Pro
196 200 205 210
Gly Ala Pro Asn Ala Pro Thr His Pro Ala Pro Pro Gly Leu His
211 215 220 225
His His His Lys His Lys Gly Gln His Arg Gln Gly His Pro Glu
226 230 235 240
Asn Arg Asp Met Pro Ala Ser Glu Asp Leu Gln Asp Leu Gln Lys
241 245 250 255
Lys Leu Cys Arg Lys Arg Cys Ile Asn Gln Leu Leu Cys Lys Leu
256 260 265 270
Pro Thr Asp Ser Glu Leu Ala Pro Arg Ser Xaa Cys Cys His Cys
271 275 280 285
Arg His Leu Ile Phe Glu Lys Thr Gly Ser Ala Ile Thr Xaa Gln
286 290 295 300
Cys Lys Glu Asn Leu Pro Ser Leu Cys Ser Xaa Gln Gly Leu Arg
301 305 310 315
Ala Glu Glu Asn Ile Thr Glu Ser Cys Gln Xaa Arg Leu Pro Pro

316 320 325 330
Ala Ala Xaa Gln Ile Ser Gln Gln Leu Ile Pro Thr Glu Ala Ser
331 335 340 345
Ala Ser Xaa Arg Xaa Lys Asn Gln Ala Lys Lys Xaa Glu Xaa Pro
346 350 355 360
Ser Asn
361

<210> 2
<211> 103
<212> PRT
<213> Homo sapiens
<220>
<223> Xaa represents selenocysteine
<400> 2
Lys Arg Cys Ile Asn Gln Leu Leu Cys Lys Leu Pro Thr Asp Ser
1               5                   10                  15
Glu Leu Ala Pro Arg Ser Xaa Cys Cys His Cys Arg His Leu Ile
16 20 25 30
Phe Glu Lys Thr Gly Ser Ala Ile Thr Xaa Gln Cys Lys Glu Asn
31 35 40 45
Leu Pro Ser Leu Cys Ser Xaa Gln Gly Leu Arg Ala Glu Glu Asn
46 50 55 60
Ile Thr Glu Ser Cys Gln Xaa Arg Leu Pro Pro Ala Ala Xaa Gln
61 65 70 75
Ile Ser Gln Gln Leu Ile Pro Thr Glu Ala Ser Ala Ser Xaa Arg
76 80 85 90
Xaa Lys Asn Gln Ala Lys Lys Xaa Glu Xaa Pro Ser Asn
91 95 100

<210> 3

<211> 33

<212> PRT

<213> Homo sapiens

<220>

<223> Xaa represents selenocysteine

<400> 3

Lys Arg Cys Ile Asn Gln Leu Leu Cys Lys Leu Pro Thr Asp Ser

1               5                   10                  15

Glu Leu Ala Pro Arg Ser Xaa Cys Cys His Cys Arg His Leu Ile

16 20 25 30

Phe Glu Lys

31

<210> 4

<211> 29

<212> PRT

<213> Homo sapiens

<220>

<223> Xaa represents selenocysteine

<400> 4

Lys Arg Cys Ile Asn Gln Leu Leu Cys Lys Leu Pro Thr Asp Ser

1               5                   10                  15

Glu Leu Ala Pro Arg Ser Xaa Cys Cys His Cys Arg His Leu

16 20 25

<210> 5

<211> 28

<212> PRT

<213> Homo sapiens

<220>

<223> Xaa represents selenocysteine

<400> 5

Thr Gly Ser Ala Ile Thr Xaa Gln Cys Lys Glu Asn Leu Pro Ser

1               5                   10                  15

Leu Cys Ser Xaa Gln Gly Leu Arg Ala Glu Glu Asn Ile

16              20                  25

## Description

### TECHNICAL FIELD OF THE INVENTION

The present invention, belonging to the field of a medical drug, relates to a novel use of a plasma protein. More specifically, the present invention relates to a medicament for treating neurodegenerative and myodegenerative diseases in association with the central and peripheral nervous systems involved in neurotransmission. Still more specifically, the present invention relates to a medicament for ameliorating neurotransmission dysfunction diseases, in particular, a medicament having an ameliorating activity to synaptic transduction, behavior of an acetylcholine receptor, and neuronal activation by nitrogen monoxide, said medicament comprising as a main active ingredient a selenocysteine-containing protein such as Selenoprotein P, a sort of plasma proteins, preferably a C-terminal peptide of said Selenoprotein P or a series of said peptides.

### BACKGROUND OF THE INVENTION

In the neural network, junctions between neurons and between neurons and effecter cells, e.g. muscular cells, are called synapse. Synapses are important for informational conduction within the neural network wherein the terminal ends of neuronal axons normally serve as an output for information (presynaptic cells) while the dendrites and the nerve cell bodies serve as an input for information (postsynaptic cells or membranes). When signal reaches at the terminal end of neurons, synaptic vesicles in the presynaptic cells are caused to open to secrete and release neurotransmitters stored therein into synaptic cleft so that neurotransmitters are bound to receptors thereof on the surface of the postsynaptic membranes to thereby conduct information to the subsequent neurons (see e.g. "Cerebral Nerve Science Illustrated", ed. by Mori et al., Yodosha).

Neurotransmitters include acetylcholine, glutamic acid, aspartic acid, γ-aminobutyric acid (GABA), glycine, serotonin, dopamine, noradrenalin, adenosine triphosphate (ATP), various neuropeptides, and the like. For instance, acetylcholine is synthesized within the living body from choline and acetyl CoA through action of choline acetyltransferase and stored in the synaptic vesicles. Such neurons as releasing acetylcholine are called cholinergic neurons. In the central nervous system, projection from the basis of forebrain to the cerebral cortex and hippocampus, projection from the pedunculopontine and laterodorsal tegmental nuclei of brainstem to the cerebral cortex, projection from interneurons in the striate body and the vestibular nuclei to the cerebellum, or motor neurons descending from the spinal cord consists of cholinergic neurons. In the peripheral nervous system, primary neurons of the sympathetic nerve, primary and secondary neurons of the parasympathetic nerve, and motor neurons all secrete acetylcholine at the terminal end thereof (see e.g. "Cerebral Nerve Science Illustrated", ed. by Mori et al., Yodosha).

On the other hand, an acetylcholine receptor on the postsynaptic membrane that receives information is largely classified into two types, i.e. muscarinic and nicotinic. A muscarinic receptor, belonging to a seven-transmembrane receptor family, mediates signal transduction towards the interior of cells via G protein. A muscarinic receptor has five subtypes based on homology and is classified into two types depending on types of G protein, i.e. one that activates phospholipase C (M1, M3, M5) and the other that inhibits adenylate cyclase (M2, M4). The former induces excitement while the latter induces restraint in cells. These receptors distribute widely not only in the brain in general but also in the heart, smooth muscle and the exocrine gland tissue. A nicotinic receptor is an ionic channel consisting of five subunits, i.e. two α subunits, and each one β, ε and δ subunits. At least nine and four subtypes are known for α and β subunits, respectively. A nicotinic receptor is classified into a skeletal muscle-type and a nerve-type depending on their distribution (see e.g. "Cerebral Nerve Science Illustrated", ed. by Mori et al., Yodosha).

Other neurotransmitters than acetylcholine also have their corresponding receptors that exhibit specific distribution within the nervous tissue. By proper transfer of these neurotransmitters through synaptic cleft, the receptors are activated, and subsequently second messengers are activated to thereby invoke physiological reactions of neurons. The reactions are transmitted either in favor of excitement (initiation of new action potential) or restraint (restraint of occurrence of action potential) of cells as controlled by the receptors, which are complicatedly intertwined together to operate the neural network within the living body (see e.g. "Cerebral Nerve Science Illustrated", ed. by Mori et al., Yodosha).

On the other hand, nitrogen monoxide (NO) is thought to be a kind of neurotransmitters as being released from the neuronal end of the autonomic nervous system and having various actions such as induction of relaxation of smooth muscle in effecter organs, control of blood flow in the brain, and erection of spongy part of penis, though no specific receptor thereof has been detected (see e.g. "Standard Physiology", 5th ed., supervised by Hongo et al., IGAKU-SHOIN Ltd.). The action of NO as intercellular signal transmitters may directly be transferred through the cellular membrane but not through any receptor or a transporter and hence may be widely spread. NO induces activation of soluble guanylate cyclase, a synthetase of a cyclic GMP (cGMP), and a synthesized cGMP in return activates a cGMP dependant phosphoenzyme to thereby trigger intracellular physiological actions to activate cells (see e.g. "Physiological Action of NO and Diseases", Ed. by Taniguchi et al., Yodosha). On the other hand, NO is considered to be a control factor for synapse flexibility since it is released from the postsynaptic cells and serves as a reverse signal transmitter to regulate release of neurotransmitters from the terminal end of the presynaptic cells (see e.g. "Physiological Action of NO and Diseases", Ed. by Taniguchi et al., Yodosha).

Abnormality in these neurotransmitters associated with neurotransmission may induce a variety of diseases. For instance, signal transduction system by acetylcholine may be involved in function of memory and learning, and function of autonomic neurons, motor neurons, sympathetic and parasympathetic neurons (see e.g. "Cerebral Nerve Science Illustrated", ed. by Mori et al., Yodosha) and hence abnormality in signal transduction mediated by acetylcholine will cause disorders in these functions that may be the cause of various diseases. For diseases associated with defect in neurotransmission, various diseases are known, including for instance Alzheimer disease, anxiety, autism, brain disturbance, depression, Huntington chorea, mania, pain, parkinsonism, etc. as a disease caused by unbalanced neurotransmitters; myasthenia gravis, Slow-channel congenital myasthetic syndrome, amyotonia congenita, etc. as a disease with defect in a receptor of neurotransmitters; amyotrophic lateral sclerosis as a disease caused by decreased intake of neurotransmitters by neurons; paroxysmal ataxia, hyperkalemic periodic paralysis, hypokalemic periodic paralysis, Lambert-Eaton syndrome, congenital paramyotonia, rasmussen encephalitis, spinocerebellar degenerative disease, etc. as a disease with defect in ion channels to disturb normal neurotransmission; botulism, intoxication by snake venom, etc. as a toxic disease (see e.g. "Web site Merck Manual, 17th. ed. in Japanese" http://www.merckmanual.banyu.co.jp/; and "How to Carry Out Cerebral Nerve Study", ed. by Manabe et al., Yodosha).

A medicament has been developed that ameliorates neurotransmission dysfunction in the autonomic nervous system and pathological conditions associated therewith. For instance, as a medicament for glaucoma for decreasing ocular tension, acetylcholine analogues such as pilocarpine and carbachol are known (see e.g. "Grand Medical Dictionary" CD-ROM, NANZANDO). Also, a medicament that stimulates a muscarinic receptor on the salivary gland to promote salivary secretion and an enterokinesis activator accompanied by an acetylcholine release-promoting activity have been developed as a drug for treating functional gastroenteritis (see e.g. New Current, 26, 13,2002).

### DISCLOSURE OF THE INVENTION

### (Technical Problem to be Solved by the Invention)

Most of the conventional drugs for treating the diseases mentioned above are one for inhibiting enzymatic degradation or reabsorption of neurotransmitters to thereby prolong their half-life, such as e.g. neurotransmitters, their agonist, antagonist or an anticholinesterase, each targeting a direct reaction between neurotransmitters and their receptors (see e.g. New Current, 2, 7,1996). However, as described above, neurotransmitters and their receptors have many types and diverse actions such as excitement and restraint to cells and thus adverse side effects tend unexpectedly to occur. For instance, administration of an excess amount of anticholinesterase may cause cholinergic crisis (drastic paralytic symptom in muscles) or its longterm administration may accelerate alteration of the receptor to aggravate diseased conditions (see e.g. "myasthenia gravis" http://www.nanbyou/tokuteisikkan/s/si7. html). Besides, since neurotransmission is profoundly associated with the autonomic nervous system, there is no denying that fatal dysfunction of the heart and the lung may occur. In order to obviate these adverse side effects, there is a need for a drug that has a different action mechanism from that of the conventional drugs and is safe with less adverse side effects.

### (Means for Solving the Problems)

Under the circumstances, the present inventors have previously found that Selenoprotein P (hereinafter also referred to as "SeP"), which is a protein derived from blood components and is a kind of selenocysteine-containing proteins, and preferably a C-terminal peptide of Selenoprotein P exhibit a cell-death inhibitory activity which hitherto has not been reported and based on this finding have filed a patent application (see e.g. PCT/JP99/06322). The present inventors further investigated to develop a medicament for ameliorating neurotransmission dysfunction diseases, in particular, a medicament having an ameliorating activity to synaptic transduction, behavior of an acetylcholine receptor, and neurotransmission by nitrogen monoxide. As a result, the present inventors surprisingly have found that Selenoprotein P, a C-terminal peptide of Selenoprotein P and a series of the C-terminal peptides as described above, which skilled artisan have not attempted to investigate, exhibit not only a cell-death inhibitory activity but also an activity to ameliorate neurotransmission function by a culture experiment with neurons and actual in vivo administration into model animals and based on this finding have completed the present invention.

Selenoprotein P has been identified in 1977 as a selenium-containing protein distinct from gulutathion-peroxidase and in 1982 it was revealed that selenium was incorporated in the form of Selenocystein. In 1991, cDNA of rat Selenoprotein P was cloned to determine a full-length amino acid sequence where it was suggested that said protein may contain at most ten selenocysteines (see e.g. Hill K. E. and Burk R. F., Biomed Environ Sci., 10, p.198-208, 1997).

In 1993, nucleic acid base and amino acid sequences of human Selenoprotein P were reported (see e.g. K. E. Hill et al., Proc. Natl. Acad. Sci. USA, 90, 537, 1993). Function of Selenoprotein P was scarcely known. Recently, however, an activity to reduce phospholipid hydroperoxide (see e.g. Y. Saito et al., J. Biol. Chem. 274, 2866, 1999) or an activity to scavenge peroxynitrite (see e.g. G. E. Arteel et al., Biol. Chem., 379, 1201, 1998) have been reported in in vitro system. There are also reports that it specifically transports Se to the brain at deficiency of Se (see R. F. Burk et al., Am. J. Physiol., 261, E26-E30, 1991) and that it acts as a survival promoting factor for neurons (see J. Yan and J. N. Barrett, J. Neurosci., 18, 8682, 1998) to suggest its relationship with survival of neurons. From these two reports, however, it would be difficult to infer any other specific actions to neurons of Selenoprotein P than its activity to maintain survival of neurons. Much less there has been no report as to a finding of an activity to activate neurons or an activity involved in neurotransmission function of Selenoprotein P as in the present invention.

As a concrete embodiment, the present inventors have found that when neuron-like cells, NG108-15 cells are differentiated into neurons, addition of Selenoprotein P to culture medium enhanced complexity in development of neurite and varicosity and accelerated synaptic formation. The present inventors have further found that Selenoprotein P aggravated epileptic symptoms and increased the action of an acetylcholine receptor in model mice for epileptic induction using a muscarinic agonist, pilocarpine. Moreover, the present inventors have found that when mouse primary neurons are cultured to generate nitrogen monoxide at a concentration not affecting the neurons, the presence of Selenoprotein P in the culture accelerated mitochondrial function of neurons to thereby activate the neurons. These indicated that Selenoprotein P had an activity to accelerate the synaptic formation, the function of an acetylcholine receptor and the activation of neurons by NO, namely an activity to ameliorate the neurotransmission function where neurons are involved.

Based on the findings as described above, the present invention relates to a novel pharmacological efficacy of Selenoprotein P and as such Selenoprotein P is an active ingredient of a medicament for ameliorating neurotransmission dysfunction diseases of the present invention. More specifically, the present invention is characteristic of selenocysteine contained in Selenoprotein P as containing selenium and this amino acid plays a key role in the ameliorating activity to neurotransmission, in particular, to synaptic transduction, behavior of an acetylcholine receptor, and neurotransmission by nitrogen monoxide. The present inventors have disclosed in the previous patent application that C-terminal peptide fragments of Selenoprotein P, a protein derived from blood components, had a cell-death inhibitory activity, which activity has not hitherto been reported, and that selenocysteine was involved in said activity. It is apparent that selenocysteine contained in Selenoprotein P is involved in the activity according to the present invention. Accordingly, a protein and/or peptide(s) that contains selenocysteine and has a cell-death inhibitory activity may be a candidate for a medicament for ameliorating neurotransmission dysfunction diseases.

Selenium per se, as may be involved in the present invention, is one of essential trace elements and it is known that its deficiency may induce a serious deficiency disease accompanied by e.g. cardiomyopathy. It is also demonstrated that selenium is essential for survival, maintenance of life or growth of cells, seeing that addition of sodium selenite to culture medium is indispensable during serum-free culture. However, as will be understood from the fact that selenium compounds are designated as poisonous substance, a range from effective to toxic amounts, i.e. a safety range of concentration, is narrow and hence selenium compounds when used in such an amount that exceeds an acceptable amount may be toxic to cells to induce unfavorably cell death. Acute toxic symptoms of selenium include, for instance, pale face, neurological symptoms, dermatitis, and gastrointestinal disorders. In addition, selenocystine, i.e. a dimer of selenocysteine, exhibits considerably high toxicity when added alone to cell culture. On the contrary, no such a high toxicity could be observed in Selenoprotein P or a C-terminal fragment of Selenoprotein P as a preferable embodiment of the present invention in spite of their containing 9 to 10 selenocysteine residues. Selenoprotein P, as naturally occurring in blood, circulates within the living body and hence is believed to be highly safe for use as a medicine. From this point of view, it is crucial that Selenoprotein P as an active ingredient for exerting the pharmacological efficacy according to the present invention contains selenocysteine and has a decreased toxicity.

### (More Efficacious Effects than Prior Art)

The peptide and a series of said peptides of the present invention not only solves the problems associated with selenium compounds, i.e. decrease in toxicity, but also allows for providing an ameliorating activity to neurotransmission that is unforeseeable to skilled artisan.

In accordance with the present invention, a medicament for ameliorating neurotransmission dysfunction diseases suitable for treating diseases with abnormality in neurotransmission function, in particular, an ameliorating agent to synaptic transduction, an ameliorating agent to behavior of an acetylcholine receptor, and an ameliorating agent to neurotransmission by nitrogen monoxide are provided.

### BRIEF DESCRIPTION OF DRAWINGS

Fig. 1 shows an action of Selenoprotein P to accelerate synaptic formation. Three days after differentiation, Lab3 antibody dyeing/simultaneous photographing with transmitted light wherein portions dyed with Lab3 antibody are bright in white.
Fig. 2 shows a change with lapse of time in survival rates after challenge with pilocarpine.
Fig. 3 shows a cell-activating activity by coordination of Selenoprotein P and Selenoprotein P fragments with nitrogen monoxide.

### BEST MODE FOR CARRYING OUT THE INVENTION

A selenocysteine-containing protein as used herein may be any protein in any molecular form as far as it contains selenocysteine and has a desired ameliorating activity to neurotransmission. Namely, the present invention encompasses a complete molecule of Selenoprotein P (SEQ ID NO: 1) as well as any other diverse protein derived from Selenoprotein P in various molecular forms. Among these, a C-terminal peptide of Selenoprotein P or a series of said peptides is most preferable. Especially, a peptide having the amino acid sequence consisting of 103 amino acid residues at the C-terminal of Selenoprotein P (SEQ ID NO: 2, from 260th to 362nd amino acids in the sequence of Selenoprotein P: 260KRCINQLLCKLPTDSELAPRSUCCHCRHLIFEKTGSAITUQCKENLPSLCSUQGLR AEENITESCQURLPPAAUQISQQLIPTEASASURUKNQAKKUEUPSN362), or a peptide having said amino acid sequence with one or several amino acid residues therein being deleted, substituted or added, or a peptide having a partial sequence of either of the above amino acid sequences, or a peptide having an amino acid sequence comprising as a part any of the above amino acid sequences, and a series of said peptides are most recommendable as a preferable embodiment.

"A series of said peptides" as used herein refers to assemblage of peptides with any amino acid sequence that contain selenocysteine and has a desired ameliorating activity to neurotransmission, and preferably assemblage of peptides with an amino acid sequence derived from that of Selenoprotein P that contains at least one selenocysteine wherein one or several amino acid residues are deleted, substituted or added and has minor structural differences due to the presence or absence of glycosylation, difference in electric charge, diversity in fragmentation, and the like. Namely, Selenoprotein P and a series of said peptides according to the present invention may be those with an amino acid sequence derived from that of a selenocysteine-containing protein, especially Selenoprotein P, that has a cytotoxicity-inhibitory activity in any molecular form, including complete Selenoprotein P as well as C-terminal peptides of Selenoprotein P. The peptides according to the present invention may be prepared with a peptide synthesizer in a conventional manner. It may also be used as a leading material for designing synthetic chemical compounds.

Selenoprotein P or a peptide derived from Selenoprotein P or a series of said peptides for use in the present invention may be prepared by any known technique, e.g. by isolation from human blood or by the genetic recombination technique. Selenoprotein P or a peptide derived from Selenoprotein P or a series of said peptides for use in the present invention as an active ingredient in a medicament for ameliorating neurotransmission dysfunction diseases is more stable to heat, a denaturing agent, a broad range of pH, or proteases in blood than normal enzymes. Thus, in one embodiment, they may be purified and identified from plasma using a fractionation with a variety of applicable carriers, including a variety of chromatographic processes such as heparin chromatography, cation exchange chromatography, anion exchange chromatography, hydrophobic chromatography, gel filtration chromatography, reverse phase chromatography, hydroxyapatite chromatography, affinity chromatography, e.g. affinity chromatography with antibody column, as well as other fractionation methods such as ammonium sulfate fractionation, molecular weight membrane fractionation, isoelectric fractionation, electrophoretic fractionation, etc. A combination of any of these fractionation methods allows for isolation of desired Selenoprotein P or a peptide derived from Selenoprotein P or a series of said peptides. Preferable combinations are exemplified in Preparations 1 and 2.

In accordance with the present invention, said protein or peptide or a series of said peptides as an active ingredient may be combined with an appropriate carrier or filler known in the art in a conventional manner to formulate a medicament for ameliorating neurotransmission dysfunction diseases of the present invention. An effective dose of a medicament for ameliorating neurotransmission dysfunction diseases of the present invention may vary depending upon age, symptoms or severity of subject to which the medicament is to be administered and ultimately upon physician's discretion. Pharmaceutical efficacy will not depend upon a route of administration but subcutaneous, intradermal, intraperitoneal, single (bolus) intravascular administration or instillation is most suitable. In case of peptides with smaller molecular weight, oral or transdermal administration may also be applied.

### INDUSTRIAL APPLICABILITY

A medicament for ameliorating neurotransmission dysfunction diseases of the present invention may be applied to any disease that is caused by abnormality or deficiency in signal transduction between neurons or between neurons and effecter cells such as muscle and is accompanied by neuropsychiatric symptoms, or symptoms of ataxia or autonomic imbalance, and the like. Neurotransmission dysfunction diseases include, for instance, diseases caused by abnormality in synaptic formation, abnormality in function of an acetylcholine receptor, or abnormality in neurotic activity by NO, including e.g. Alzheimer disease, anxiety, autism, brain disturbance, depression, Huntington chorea, mania, pain, parkinsonism, myasthenia gravis, Slow-channel congenital myasthetic syndrome, amyotonia congenita, amyotrophic lateral sclerosis, paroxysmal ataxia, hyperkalemic periodic paralysis, hypokalemic periodic paralysis, Lambert-Eaton syndrome, congenital paramyotonia, rasmussen encephalitis, spinocerebellar degenerative disease, botulism, intoxication by snake venom, and the like. The diseases further include glaucoma, diseases where promotion of salivary secretion is required, or functional gastroenteritis where activation of enterokinesis is needed. Another diseases that may be encompassed by the present invention are dementia or dyskinesia associated with aging. A medicament for ameliorating neurotransmission dysfunction diseases comprising as an active ingredient Selenoprotein P or a peptide derived from Selenoprotein P or a series of said peptides of the present invention may be applied alone or may be combined with other drugs for further potentiating efficacy of said medicament. It is expected that the medicament according to the present invention may efficaciously be applied both for prophylaxis and treatment of diseases.

The present invention is explained in more detail by means of the following Preparations and Examples but should not be construed to be limited thereto. Reagents used in the following Preparations and Examples were from Wako Pure Chemical Industries, Ltd., TAKARA SHUZO CO., Ltd., Toyobo, New England BioLabs, Amersham Bioscience, BioRad, Sigma and Gibco BRL. unless otherwise mentioned. Selenoprotein P and its fragments were prepared in Preparations for use in Examples.

### Preparation 1

### (Purification of Selenoprotein P Fragments)

A heparin Sepharose-binding fraction from plasma was precipitated with 2 M ammonium sulfate. The precipitate was dissolved in more than 5 volumes of 20 mM Tris buffer, pH 8.0. Selenoprotein P in this solution was adsorbed to anti-SeP antibody column and the carrier was washed with PBS. Selenoprotein P was then eluted with 20 mM citrate buffer, pH 4.2, containing 4 M urea and was adsorbed to a cation exchanger (Macroprep High S: BioRad) equilibrated with 20 mM citrate buffer, pH 4.2. Then, gradient elution was performed with a salt concentration of sodium chloride and fraction with the cell death-inhibitory activity was recovered. At this point, a full-length Selenoprotein P could also be obtained but with a cell death-inhibitory activity per proteins being much lower than that of the fragment thereof. According to the procedures as described herein, purification may be carried out in a short time and hence Selenoprotein P fragments with higher cell death-inhibitory activity per proteins could be obtained. The fragments obtained here were also a fraction of a mixture containing various molecular species with various sizes depending upon the presence or absence of glycosylation, intermolecular bonding, or inner cleavage, etc. They were an assemblage of Selenoprotein P fragments that showed a size ranging from 10 to 30 kDa in electrophoresis under non-reductive condition.

### Preparation 2

### (Purification of Selenoprotein P)

To human plasma were added diisopropyl fluorophosphate (Wako Pure Chemical Industries, Ltd.) and polyethylene glycol 3000 (SIGMA) at a final concentration of 2 mM and 5%, respectively. The mixture was stirred for 1 hour and centrifuged at 10,000 rpm for 15 minutes to recover a supernatant. The obtained supernatant was bound to anti-Selenoprotein P antibody column equilibrated with PBS and the column was washed with PBS. Selenoprotein P was then eluted with 20 mM citrate buffer, pH 4-6, containing 4 M urea and was adsorbed to a cation exchanger (Macroprep High S: BioRad) equilibrated with 20 mM citrate buffer. Then, gradient elution was performed with a salt concentration of sodium chloride and a fraction with the highest content of selenium was recovered. This process generated around 2 mg of a full-length Selenoprotein P with a molecular weight of 64 kDa from 1 liter of plasma after electrophoresis under reduced condition.

### Example 1

### (Activity to accelerate synaptic formation)

In order to study the effect of Selenoprotein P on synaptic formation, differentiation to neurons was examined using NG108-15 cells (hybridoma of rat neuroblastoma and rat glioma: ATCC NO. HB-12317) which has widely been used for study of synaptic formation and a receptor. NK108-15 cells were seeded to DMEM supplemented with 10% fetal calf serum and HAT (hypoxanthine, aminopterin and thymidine) in 35mm polyornithine-coated dish and incubated at 37°C, 10% CO₂. After 1 to 2 days, the culture medium was replaced with DMEM (serum free medium) supplemented with 1% fetal calf serum and HT (hypoxanthine and thymidine) and 0.1 mM dibutyl cyclic AMP (abcAMP) was added to induce differentiation while Selenoprotein P at 4.36 µg/mL was added to the medium followed by culture for 3 days. After culture, the dish attached with a sheet of cells was rinsed twice with phosphate buffered saline (hereinafter referred to as PBS) to wash the culture. Cellular proteins were then fixed with paraformaldehyde adjusted to 4% with PBS. After fixation at room temperature for 30 minutes, the dish was rinsed twice with PBS to remove a fixing solution. Then, mouse anti-Rab3a monoclonal antibody (Synaptic Systems) reactive with Rab3a, a protein expressed specifically at synapse of neurons, was adjusted to an appropriate concentration with PBS and added together with 10% bovine serum albumin for blocking non-specific proteins and the mixture was reacted at room temperature for 1 hour (primary antibody reaction). After completion of the antibody reaction, the dish was rinsed with PBS three to four times and anti-mouse IgG antibody cross-linked with fluorescent probe Alexa 594 was reacted at room temperature for 1 hour (secondary antibody reaction). After completion of the antibody reaction, the dish was rinsed with PBS three to four times and then observed for red fluorescent image with a laser microscopy at 594 nm of an excitation wave-length and photographed with a digital camera.

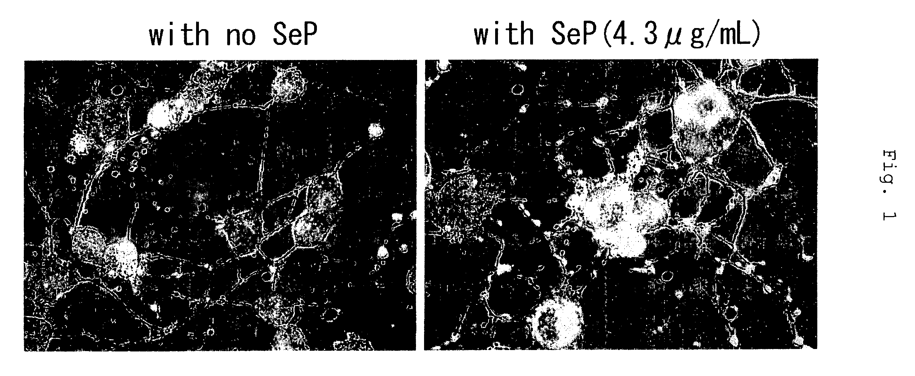

As a result of culture, it was observed that the cells induced for differentiation with selenoprotein for 3 days apparently exhibited complexity in development of neurites as well as much particulate varicosity. The nerve cell bodies had also a number of short neurites. On the other hand, the cells cultured in the absence of selenoprotein were generally round in their shape and had poor varicosity though some had long neurites. When dyed with mouse synapse-specific anti-Rab3a monoclonal antibody, the cells cultured with Selenoprotein P exhibited a large number of Rab3a-dyed knotty particles on neurites as shown in Fig. 1. A proportion of synapses specifically dyed with the anti-Rab3a antibody was then calculated with Mac SCOPE (manufactured by Mitani Corporation), a software for image analysis for Mackintosh, to measure immunopositive spots. Four and three visions of photographed pictures were randomly selected for the cells with and without SeP, respectively, and used for analysis. The nerve cell bodies portions in the photographed pictures were excluded from selection and the neurites portions were adopted for analysis. On the digital image, immunopositive portions have a high value in Red (R) in the level of the three primary colors (RGB). Therefore, those spots with R value alone could be extracted with the analysis software and measured. In addition, the number of varicosity with no immunological reaction was measured manually and then a proportion (%) of the number of immunopositive varicosity among a total number of varicosity in the photographed picture was calculated. As a result, it was proved that synaptic formation was apparently accelerated as shown in Table 1.

**Table 1**

| | Proportion of Rab3a-immuno-positive cells ± SD (%) |
|---|---|
| Cells cultured with SeP | 66.0 ± 2.75 |
| Cells cultured without SeP | 38.8 ± 13.3 |

### Example 2

### (Activity to accelerate function of acetylcholine receptor)

In order to study the effect of Selenoprotein P on cholinergic neurons, to mice was administered pilocarpine that agonistically acted on acetylcholine receptors present on junctions (synaptic terminal) between neurons and between neurons and muscle to thereby induce convulsion. Pilocarpine, having a parasympathetic nervestimulating activity, will induce clonic convulsion in the limbs leading to systemic convulsion when administered to mice.

Twelve ICR male mice (weighing 30 to 43 g; purchased from CLEA Japan, Inc.) of nine weeks old were divided into two groups (6 animals/group) and tested. One hour before administration of pilocarpine, Selenoprotein P prepared at a concentration of 2.5 mg/mL with saline was administered to mice at 0.5 mg of Selenoprotein P per animal (200 µL) as a test group. For a control group, an equivalent amount of saline was administered intraperitoneally. Pilocarpine (pilocarpine hydrochloride, Wako Pure Chemical Industries, Ltd.) was prepared at 100 mg/mL with saline and administered to mice intraperitoneally at 270 to 320 mg/kg. Immediately after administration, mice were photographed with a digital video for record of experiment. Observation was made for 60 minutes after administration of pilocarpine. Time required for developing systemic convulsion and survival of the animals were taken for criteria of assessment. If Selenoprotein P will enhance the activity of pilocarpine, then time required for paroxysm will be shortened while mortality will increase due to acceleration of convulsive symptoms.

As a result of this experiment, mice administered with Selenoprotein P exhibited significantly shortened time for developing systemic convulsion as compared to that of a control group to prove that administration of Selenoprotein P enhanced induction of convulsion in mice as shown in Table 2.

**Table 2**

| | Time required for developing convulsive attack after admin. of pilocarpine ± SD (%) |
|---|---|
| Mice admin. with SeP | 6 min. 47 sec. ± 40 sec.* |
| Mice not admin. with SeP | 12 min. 29 sec. ± 300 sec.* |

| | |
|---|---|
| *p < 0.05 | |

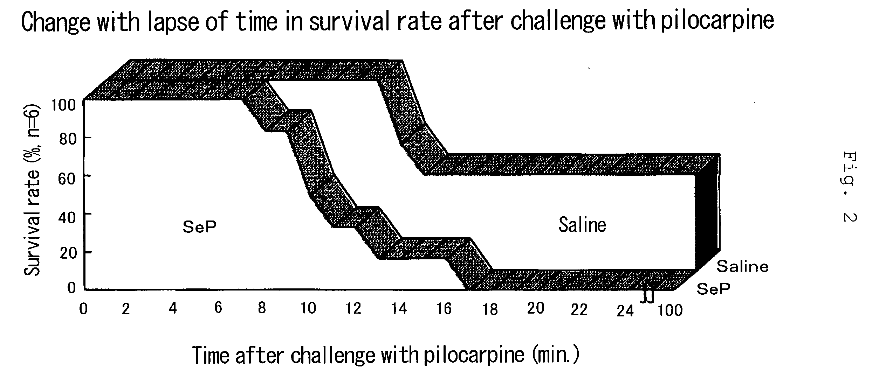

Similarly, it was proved that mice administered with Selenoprotein P had apparently decreased mortality than that of a control group as shown in Fig. 2. As a result of this experiment, possibility was suggested that Selenoprotein P enhanced the activity of pilocarpine on muscarinic acetylcholine receptors.

### Example 3

### (Activity to accelerate activation of neurons by NO)

In order to study the effect of Selenoprotein P on the neurotic activity of NO as synaptic neurotransmitter, cultured primary neurons were subject to the action of an NO generator, S-nitroso-N-acetyl-DL-penicillamine (SNAP, manufactured by DOJINDO LABORATORIES), in the presence of Selenoprotein P to determine as to whether a respiratory capacity of neuronal mitochondria is altered in the presence of Selenoprotein P. For determination of a respiratory capacity of mitochondria, a kit was used that readily measured the dehydrogenase activity in the mitochondrial inner membrane (Cell Counting kit-8, manufactured by DOJINDO LABORATORIES).

Fetus was removed from C57BL/6 mouse of 14-day pregnancy (purchased from Charles River Japan, Inc.). The cerebral cortex from the fetus was treated and dispersed with 0.25% trypsinized EDAT and then cultured in Neurobasal Medium (Gibco BRL.) supplemented with B27 supplement (Gibco BRL.) in 5% CO₂ incubator at 37°C for culture of primary neurons. For culture, a 48-well multi-well culture plate, previously coated with poly-D-lysine at 4 µg/cm², was used and thereto were inoculated the neurons at a density of 2 × 10⁵ cells/well. The neurons were cultured in 500 µL/well while a half of the culture medium was replaced with a fresh medium every 2 to 3 days to maintain the neurons for 11 days. On Day 11, the culture medium was removed and replaced with Neurobasal Medium alone. The culture was divided into two groups with and without SNAP at 50 µM. Each group was further divided into groups where either Selenoprotein P or sodium selenite at 1 µM or Selenoprotein P fragment at 0.26 µM as a concentration of selenium was added. The SNAP concentration of 50 µM was set so as not to damage neurons. After culture in 5% CO₂ incubator at 37°C for 15 hours, a one tenth equivalent of the reaction of Cell Counting kit-8 to the culture was added and the mixture was reacted in 5% CO₂ incubator at 37°C for 4 hours. After developing reaction, each 100 µL of the culture supernatant was transferred to a 96-well microtiter plate and then absorption was measured at a wave-length of 450 nm with a microtiter plate reader with a reference wave-length of 650 nm.

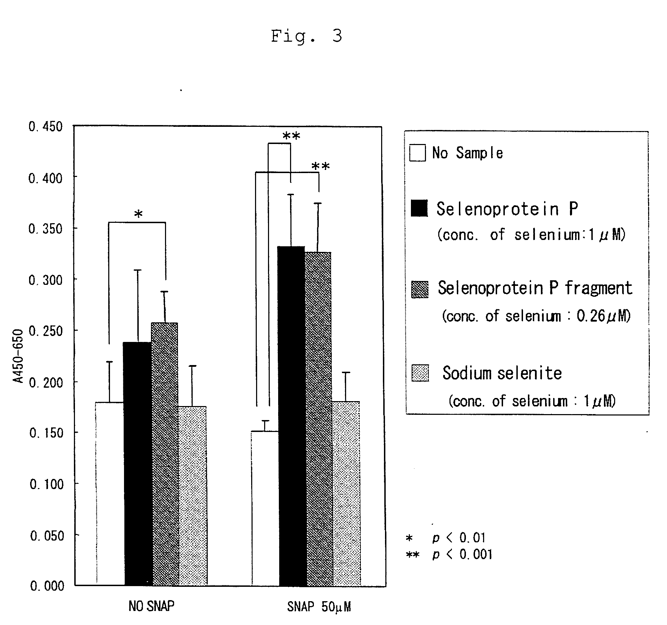

As a result of this experiment, with addition of Selenoprotein P alone, the primary neurons tended to show an increased activity of dehydrogenase of the mitochondrial inner membrane (mitochondrial respiratory capacity) as shown in Fig. 3. Similarly, in the group where Selenoprotein P fragment alone was added, the cellular activity of the primary neurons was significantly increased. In the presence of an NO generator, i.e. SNAP, the mitochondrial respiratory capacity of the neurons was significantly increased with either addition of Selenoprotein P or Selenoprotein P fragment. On the contrary, as not being detected in the group where sodium selenite was added, this activity was thought to be specific for Selenoprotein P or Selenoprotein P fragment. Since it is believed that primary neurons do not proliferate (i.e. not subject to cell division), this increase in the enzymatic activity may result from the intracellular activation but not from increase in the number of neurons. As such, Selenoprotein P and Selenoprotein P fragment have an activity to accelerate the intracellular, mitochondrial respiratory capacity, i.e. the cellular activity, in coordination with NO. Thus it may be through this activity that Selenoprotein P and Selenoprotein P fragment provide activation of synaptic transduction and the neural network as a whole within the living body.

## Claims

1. A medicament for ameliorating neurotransmission dysfunction diseases comprising as a main active ingredient a selenocysteine-containing protein and/or a selenocysteine-containing peptide that consists of said protein or a series of said peptides.

2. The medicament for ameliorating neurotransmission dysfunction diseases according to claim 1 wherein said selenocysteine-containing protein is Selenoprotein P.

3. The medicament for ameliorating neurotransmission dysfunction diseases according to claim 1 wherein said selenocysteine-containing peptide is a C-terminal peptide of Selenoprotein P.

4. The medicament for ameliorating neurotransmission dysfunction diseases according to any one of claims 1 to 3 wherein said C-terminal peptide of Selenoprotein P or a series of said peptides is a protein or a peptide or a series of said peptides that has either the amino acid sequence of from 260th to 362nd amino acids in the C-terminal of Selenoprotein P, or said amino acid sequence with one or several amino acid residues therein being deleted, substituted or added, or a partial sequence of either of the above amino acid sequences, or an amino acid sequence comprising as a part any of the above amino acid sequences.

5. The medicament for ameliorating neurotransmission dysfunction diseases according to claim 4 wherein said C-terminal peptide of Selenoprotein P or a series of said peptides is a peptide or a series of said peptides that has either the amino acid sequence of:
(I): Lys Arg Cys Ile Asn Gln Leu Leu Cys Lys Leu Pro Thr Asp Ser Glu Leu Ala Pro Arg Ser Sec Cys Cys His Cys Arg His Leu (SEQ ID NO: 4) and/or
(II): Thr Gly Ser Ala Ile Thr Sec Gln Cys Lys Glu Asn Leu Pro Ser Leu Cys Ser Sec Gln Gly Leu Arg Ala Glu Glu Asn Ile (SEQ ID NO: 5)
wherein Ala is alanine, Arg is arginine, Asn is asparagine, Asp is aspartic acid, Cys is cysteine, Gln is glutamine, Glu is glutamic acid, Gly is glycine, His is histidine, Ile is isoleucine, Lys is lysine, Leu is leucine, Met is methionine, Phe is phenylalanine, Pro is proline, Ser is serine, Thr is threonine, Trp is tryptophan, Tyr is tyrosine, Val is valine, and Sec is selenocysteine;
or a partial sequence of said amino acid sequence, or said amino acid sequence with one or several amino acid residues therein being deleted, substituted or added, or a partial sequence of either of the above amino acid sequences, or an amino acid sequence comprising as a part any of the above amino acid sequences.

6. The medicament for ameliorating neurotransmission dysfunction diseases according to any one of claims 1 to 5 wherein said neurotransmission dysfunction diseases are diseases caused by abnormality in synaptic formation, abnormality in function of an acetylcholine receptor, or abnormality in neurotic activity by nitrogen monoxide (also referred to as NO).

7. The medicament for ameliorating neurotransmission dysfunction diseases according to claim 6 wherein said neurotransmission dysfunction diseases are selected from myasthenia gravis, Slow-channel congenital myasthetic syndrome, amyotonia congenita, Lambert-Eaton syndrome, Alzheimer disease, dementia, spinocerebellar degenerative disease, autonomic imbalance, erection failure of spongy part of penis, failure of blood flow in the brain, functional gastroenteritis, and glaucoma.
